# EUROPÄISCHE PATENTSCHRIFT

(11) **EP 1 459 683 B1**
(45) Veröffentlichungstag und Bekanntmachung des Hinweises auf die Patenterteilung: **29.02.2012**
(21) Anmeldenummer: 04006183.0
(22) Anmeldetag: 16.03.2004
(51) Int. Cl.: A61B 5/15

(54) **Stechhilfe mit Lanzettensystem mit Wiederverwendungsschutz**
Puncturing aid with lancet device and prevention of reuse
Accessoir de piquage avec dispositif de lancettes et d' empêchement de la réutilisation

(30) Priorität: 20.03.2003 DE 10312357
(43) Veröffentlichungstag der Anmeldung: 22.09.2004
(60) Teilanmeldung: 11008583.4
(73) Patentinhaber: Roche Diagnostics GmbH, 68305 Mannheim (DE); F.Hoffmann-La Roche AG, 4070 Basel (CH)
(72) Erfinder: Kuhr, Hans-Juergen, Dr., 68219 Mannheim (DE); Weiss, Thomas, 68307 Mannheim (DE); Forster, Richard, 92269 Fensterbach (DE); Sachsenweger, Peter, 93197 Zeitlarn (DE); Ebert, Karl-Peter, 64407 Fraenkisch-Crumbach (DE)

(56) Entgegenhaltungen:
- EP-A- 0 630 609
- WO-A-91/11212
- US-A- 4 817 603
- US-A- 5 324 303
- US-A- 5 628 764

## Beschreibung

Die Erfindung betrifft ein Lanzettensystem, das in einer Stechhilfe verwendet werden kann zur Entnahme von Blut für Diagnosezwecke. Bei verschiedenen Krankheiten ist es notwendig, das menschliche Blut hinsichtlich eines darin enthaltenen Analyten zu untersuchen. In vielen Fällen reicht es dabei aus, durch Erzeugung einer kleinen Stichwunde dem Körper eine geringe Menge Blut in Form eines Blutstropfens zu entnehmen. Ein besonders wichtiger derartiger Fall ist der Diabetes, bei dem das Blut in regelmäßigen Abständen auf den Glukosegehalt untersucht werden muss. Weitere Blutuntersuchungen können zum Beispiel bezüglich der Gerinnungsparameter, Triglyzeride, HbA1c oder Laktat vorgenommen werden. Zur Erzeugung der erforderlichen Stichwunden werden üblicherweise Blutlanzettenvorrichtungen verwendet, die aus einem Stechgerät und hierzu angepassten auswechselbaren Lanzetten bestehen. In dem Gehäuse des Stechgerätes befindet sich ein Lanzettenhalter, in dem jeweils eine Lanzette auswechselbar eingesetzt werden kann. Beim Einstichvorgang wird der Lanzettenhalter von einem ebenfalls in dem Stechgerät integrierten Lanzettenantrieb der Lanzette schnell in eine Einstichrichtung bewegt, bis die Nadelspitze aus einer vom vorderen Ende des Stechgerätes vorgesehen Austrittsöffnung austritt und eine kleine Stichwunde in den Körperteil, gegen das das vordere Ende gedrückt wird, erzeugt. Danach wird der Lanzettenhalter mit der Lanzette entgegen der Einstichrichtung zurückbewegt.

Im Laufe der Zeit haben sich kleine handliche Blutentnahmegeräte, sogenannte Stechhilfen, etabliert, die vom Benutzer einfach und zuverlässig bedient werden können und einen möglichst schmerzarmen Einstich in ein Körperteil zu ermöglichen. Zur Vermeidung von Infektionen, insbesondere im Krankenhaus, sind die Lanzetten zur einmaligen Verwendung vorgesehene disposible Elemente. Nach der einmaligen Verwendung einer Lanzette wird die Lanzette nach dem Stechvorgang entnommen oder vom Gerät ausgeworfen und in den Müll verworfen. Durch die in einem solchen Fall in einem Müllcontainer freiliegende Nadel kann es bei der Beseitigung des Abfalls zu Verletzungen kommen, die zur Kontamination Dritter durch die benutzte Lanzette führen können. Eine derartige Kontamination kann unter Umständen Infektionen nach sich ziehen, so dass es in einigen Ländern Bestrebungen gibt, Blutentnahmesysteme, bei denen die Nadelspitze nach der Benutzung frei zugänglich ist, zu verbieten. Zusätzlich zu einer Verletzungsgefahr bei der Müllbeseitigung besteht darüber hinaus das Risiko einer versehentlichen Wiederverwendung einer bereits gebrauchten Lanzette. Dies ist insbesondere im Krankenhausbereich, bei dem eine Stechhilfe für mehrere Patienten verwendet wird, zu bedenken, da so durch eine Unachtsamkeit des Pflegepersonals ein Patient mit dem Blut eines vorhergehenden Patienten kontaminiert werden kann.

Neben der Verwendung der Blutlanzettenvorrichtung durch medizinisches Personal werden darüber hinaus Stechhilfen auch von Laien im sogenannten Home-Monitoring-Bereich eingesetzt. Dies gilt insbesondere für die Therapiekontrolle von Diabetikern. So wurde bei der Behandlung von Diabetikern festgestellt, dass schwerwiegende, mit dem Diabetes verbundene Schäden, wie zum Beispiel Erblinden, entscheidend vermindert werden können, wenn die Glukosekonzentration im Blut des Diabetikers häufig, bis zu fünf mal täglich, bestimmt wird und aufgrund dieser Messungen die Insulininjektion exakt eingestellt werden kann. Um derart häufige Messungen zu realisieren, werden Stechhilfen im Rahmen des Home-Monitoring-Bereiches eingesetzt, sodass der Diabetiker selbst eine Blutuntersuchung vornehmen kann. Die Anforderungen, die sich hierdurch an eine Blutlanzettenvorrichtung ergeben, sind neben einer einfachen Handhabung beim Auslösen des Stechvorgangs sowie eines schmerzarmen Einstiches auch ein einfaches Handling beim Einsetzen neuer Lanzetten sowie ein sicheres Verwerfen von bereits gebrauchten Lanzetten. Ein Wechsel der Lanzetten sollte einerseits möglichst einfach sein, andererseits aber auch ein Maximum an Sicherheit gegen ungewollte Verletzungen des Anwenders sowie Dritter gewährleisten. Hierbei ist es im Home-Monitoring-Bereich denkbar, dass eine einmal eingelegte Lanzette durch den selben Benutzer zwar mehrmals zum Stechen verwendet wird; jedoch sollte auch hier, nachdem der Benutzer sich entschieden hat, die Lanzette zu verwerfen, eine versehentliche Wiederverwendung einer bereits ausgeworfenen Lanzette verhindert werden. Darüber hinaus sollte ein Schutz vor ausgeworfenen Lanzetten insbesondere für Dritte, beispielsweise bei der Müllbeseitigung, gewährleistet sein.

Im Stand der Technik wird beim Einsetzen der Lanzette die Spitze der Nadel üblicherweise von einer Spitzenabdeckung aus Kunststoff umgeben, die eine gefahrlose Handhabung beim Einführen der Lanzette ermöglicht. Wenn die Lanzette eingesteckt ist, wird die Spitzenabdeckung entfernt, damit die scharfe Spitze der Nadel für den Stichvorgang frei liegt (US 5,628,765). Durch die freiliegende Nadelspitze besteht jedoch sowohl das Risiko einer unbeabsichtigten Verletzung als auch die Möglichkeit einer Spitzenbeschädigung. Nach einem oder mehrmaligem Stechvorgang wird die Lanzette aus der Stechhilfe entfernt. Dies kann entweder manuell geschehen, wobei jedoch das Risiko einer Verletzung an der Nadelspitze hoch ist, oder durch einen automatischen Auswurfmechanismus erfolgen.

In dem Patent EP 0 565 970 wird eine Blutlanzettenvorrichtung offenbart, bei der die Lanzette mittels einer Auswurfstange aus der Lanzettenhalterung ausgestoßen wird. Durch Betätigung eines entsprechenden Knopfes kann der Benutzer die Auswurfstange bedienen.

Des Weiteren wird in der Patentschrift US 4,442,836 ein Auswurfmechanismus beschrieben, bei dem automatisch beim erneuten Spannen der Stechhilfe die Nadel ausgelöst wird, so dass die gebrauchte Lanzette nach jedem Einstichvorgang verworfen wird. Derartige Auswerfmechanismen erfordern einen relativ hohen zusätzlichen konstruktiven Aufwand. Des Weiteren ist eine mehrfache Benutzung eines bereits eingelegten Lanzettensystems nicht möglich, welches jedoch insbesondere im Home-Monitoring-Bereich von den Kunden häufig gewünscht ist. Ein weiterer wesentlicher Nachteil des beschriebenen Standes der Technik ist darüber hinaus, dass die Nadelspitze nach dem Auswurf der Lanzette ungeschützt ist, so dass ein damit verbundenes Verletzungsrisiko, wie bereits beschrieben, besteht.

Um ein gefahrloses Entfernen der verbrauchten Lanzette zu erleichtern, werden im Stand der Technik weiterhin Blutentnahmesysteme beschrieben, die nach dem Auswurf der Lanzette ein Schutz vor der Nadelspitze gewährleisten. Insbesondere für Benutzer, die im hohen Alter oder durch Folgen der Erkrankung häufig durch eine schlechte Sehfähigkeit und zitternde Hände behindert sind, wird dies als ein wesentliches Merkmal erachtet.

Um einen Schutz vor der Nadelspitze zu realisieren, wird im Stand der Technik die Lanzette in eine Kappe der Stechhilfe integriert, so dass die Lanzette und die Gehäusekappe insgesamt eine auswechselbare disposible Einheit bilden. Derartige Konstruktionen sind in den Dokumenten EP 0595148 sowie US 4,990,154, US 5,454,828 und DE 10053974 beschrieben. Wird die Lanzette von dem Benutzer ausgeworfen, wird hierbei die Gehäusekappe über die Nadelspitze gestülpt, so dass die Lanzette anschließend von der Kappe umgeben verworfen wird. Auch wenn bei den beschriebenen Mechanismen die Nadelspitze nach dem Auswurf geschützt ist, so ist dennoch ein erneutes Wiedereinführen sowie ein erneuter Stechvorgang mittels einer bereits einmal ausgeworfenen Nadel durch einen unachtsamen Benutzer möglich. Der Benutzer ist folglich darauf angewiesen, selbst zu erkennen, dass die Nadel bereits gebraucht wurde.

Das Dokument US 4,817,603 offenbart im Rahmen einer Ausführungsform eine Stechhilfe mit einem Gehäuse, das die Antriebseinheit beinhaltet und wiederverwendbar ist und einen weiteren Geräteteil, der den Auslösemechanismus sowie die Nadel beinhaltet und als Einwegartikel ausgestaltet ist.

Ausschließlich das Dokument EP 0 630 609 offenbart einen Mechanismus, der direkt das Wiedereinführen und somit ein Wiederverwenden einer einmal ausgeworfenen Lanzette verhindert.
Die beschriebene Lanzettenvorrichtung beinhaltet eine Nadel mit einem Nadelkörper, der bei Auswurf der Nadel aus der Stechhilfe zerbricht, so dass ein erneutes Wiedereinführen der Nadel verhindert wird. Hierdurch wird der Benutzer gehindert eine kontainierte Nadel wiederzuverwenden. Nachteil des Standes der Technik ist jedoch, dass nach dem Auswurf der Nadel die Nadelspitze ungeschützt vorliegt.

Aufgabe der Erfindung ist es, vorteilhafterweise für den Bereich des Home-Monitorings eine leicht zu handhabende Stechhilfe zur Verfügung zu stellen, die das erneute Benutzen eines bereits ausgeworfenen Lanzettensystems verhindert, wobei gleichzeitig nach dem Auswurf des Lanzettensystems ein Schutz vor Verletzung an der Nadelspitze gewährleistet werden soll. Vorteilhafterweise soll eine Wiederverwendung einer Nadel eines einmal eingelegten Lanzettensystems problemlos möglich sein.

Die Aufgabe wird gelöst durch eine Stechhilfe und ein Lanzettensystem gemäß der unabhängigen Ansprüche. Bevorzugte Ausführungsformen ergeben sich aus den abhängigen Ansprüchen.

Die Erfindung betrifft ein Lanzettensystem sowie eine Stechhilfe beinhaltend das Lanzettensystem. Die Stechhilfe weist ein Gehäuse auf zum Einführen eines Lanzettensystems. Das Gehäuse beinhaltet weiterhin eine Öffnung zum Austreten einer Nadelspitze aus dem Gehäuse sowie einen Antriebsmechanismus zum Ausführen eines Stechvorgangs. Erfindungsgemäß weist das Gehäuse zusätzliche ein Halteelement auf, dass mit einem hierzu entsprechenden Halteelement des Lanzettensystems zusammenwirken kann, sobald das Lanzettensystem in die Stechhilfe eingelegt wird. Aufgrund des Zusammenwirkens der Halteelemente kann das Lanzettensystem im Gehäuse an einem definierten Ort positioniert werden. Hierbei ist eine exakte Positionierung des Lanzettensystems insbesondere hinsichtlich des Antriebsmechanismus der Stechhilfe von Bedeutung, da nur so der Antriebsmechanismus an die Nadel fehlerfrei ankoppeln kann, sodass die Nadel mit hoher Geschwindigkeit und nahezu ohne Vibrationen einen Stechvorgang ausführt. Dies ermöglicht einen schnellen und schmerzarmen Einstich in einen hierfür vorgesehenen Körperteil. Das Lanzettensystem für die Stechhilfe beinhaltet neben dem beschriebenen Halteelement mindestens eine Nadel mit einer Spitze, die zur Erzeugung einer Hautöffnung geeignet ist. Die Nadel ist mit einem Nadelkörper verbunden, wobei zumindest ein Schutzbereich des Nadelkörpers und die Nadel relativ zueinander beweglich sind. Hierbei wird die Nadelspitze in einer ersten Position zumindest teilweise vom Schutzbereich des Nadelkörpers umgeben, während in einer zweiten Position der Schutzbereich des Nadelkörpers und die Nadelspitze so zueinander angeordnet sind, dass die Nadelspitze vom Schutzbereich des Nadelkörpers freigegeben wird. Befindet sich der Schutzbereich des Nadelkörpers in seiner ersten Position, wird folglich ein Verletzungsschutz vor der Lanzettenspitze gewährleistet, der insbesondere nach dem Auswurf des Lanzettensystems aus der Stechhilfe von Bedeutung ist.

Der Nadelkörper beinhaltet weiterhin einen Sperrmechanismus, der im Zusammenwirken mit der Stechhilfe aktiviert wird. Aufgrund des Sperrmechanismus erfolgt eine Veränderung des Nadelkörpers in der Weise, dass nach dem Auswurf des Lanzettensystems aus der Stechhilfe ein Zusammenwirken des Halteelementes der Stechhilfe mit dem Halteelement des Lanzettensystems bei einem erneuten Wiedereinführen nicht mehr möglich ist. Hierdurch wird das Wiederverwenden eines einmal ausgeworfenen Lanzettensystems verhindert. Eine automatische Betätigung des Sperrmechanismus kann hierbei erfolgen, sobald bestimmte Bedienungsschritte an der Stechhilfe vorgenommen werden. Es sind jedoch auch Ausführungsformen denkbar, bei denen der Benutzer durch einen separaten Bedienungsschritt den Sperrmechanismus betätigt.

Das erfindungsgemäße Lanzettensystem ermöglicht durch die spezielle Ausgestaltung des Nadelkörpers zum einen einen Schutz gegen die Nadelspitze, so dass die Spitze auch nach dem Auswurf des Lanzettensystems von dem Schutzbereich des Nadelkörpers so weit umgeben ist, dass eine Verletzung durch die Spitze verhindert wird. Zum anderen beeinflusst der Sperrmechanismus das Zusammenwirken der Halteelemente. Im Rahmen der Erfindung umfasst dabei der Begriff Zusammenwirken der Halteelemente jegliche erdenkliche Ausführungsform, wie sie im Stand der Technik zum Einführen und Positionieren einer Lanzette oder eines Magazins in eine Stechhilfe hinlänglich bekannt sind. Beispielsweise ist hier ein Einrasten oder ein Verklemmen der Halteelemente denkbar. Hierfür geeignete Halteelemente können beispielsweise in Form von Rastnasen, Rillen oder Haken - um nur einige mögliche Ausführungsformen zu nennen-ausgebildet sein. Analog zu den im Stand der Technik beschriebenen Systemen mit Einzellanzetten, ist es darüber hinaus auch denkbar, dass durch das Ankoppeln der Antriebseinheit an das Lanzettensystem bereits eine hinreichende Positionierung und Halterung in der Stechhilfe gewährleistet wird, so dass z. B. die Antriebseinheit selbst als Halteelement für ein entsprechend ausgebildetes Lanzettensystem dienen kann.

Sind mehrere Halteelemente zur Positionierung des Lanzettensystems vorgesehen, verhindert vorteilhafterweise der Sperrmechanismus das Zusammenwirken jeglicher Halteelemente des Lanzettensystems und der Stechhilfe, so dass das Lanzettensystem in der Stechhilfe nicht gehalten und positioniert werden kann. Dies erweist sich insbesondere dann als vorteilhaft, wenn das Lanzettensystem und die Stechhilfe jeweils über mehrere, voneinander unabhängig wirkende Halteelemente verfügt.

In der bevorzugte Ausführungsform wird das Zusammenwirken der Halteelemente in der Weise verhindert, dass ein Wiedereinführen des Lanzettensystems in die Stechhilfe verhindert wird. Im Rahmen der Erfindung umfasst der Begriff "Wiedereinführen" eine Handhabung des Lanzettensystems in der Weise, dass das Lanzettensystem in der Stechhilfe an dem zum Ausführen des Stechvorgangs vorgesehenen Ort positioniert und aufgrund des Zusammenwirkens der Halteelemente gehalten wird. Hierzu wird das Lanzettensystem wieder an seinem ursprünglichen Ort in der Stechhilfe eingesetzt, so dass der Originalzustand zwischen Lanzettensystem und Stechhilfe wieder hergestellt wird, der beim erstmaligen Gebrauch der Stechhilfe vorliegt.

Bei der Handhabung der Stechhilfe kann der Benutzer vorteilhafterweise ein bereits gebrauchtes Lanzettensystem unmittelbar und eindeutig erkennen; beispielsweise dadurch, dass bereits ein Wiedereinführen des Lanzettenmagazins in die Stechhilfe blockiert wird. Anders als im Stand der Technik ist somit der Benutzer nicht aufgefordert, ein gebrauchtes Lanzettensystem von einem neuen Lanzettensystem bewusst unterscheiden zu müssen. Vorteilhafterweise bleibt dem Benutzer ein unnötiges, insbesondere häufig für ältere oder sehbehinderten Personen schwerfallendes Wiedereinführen eines nicht mehr funktionstüchtigen, gebrauchten Lanzettensystems erspart.

In einer bevorzugten Ausführungsform wird der Sperrmechanismus im Wesentlichen durch eine Formveränderung des Nadelkörpers realisiert. Dies erweist sich insbesondere als vorteilhaft, wenn die Form des Nadelkörpers selbst zumindest einen Teil eines Halteelementes ausbildet. Es ist weiterhin auch denkbar, dass aufgrund einer Formveränderung des Nadelkörpers eine räumliche Trennung der Halteelemente in der Stechhilfe resultiert, sodass der Sperrmechanismus auf diese Weise indirekt Einfluss auf ein Halteelement nimmt, ohne auf dieses direkt einzuwirken. Das Lanzettensystem kann somit nicht mehr an einem definierten Ort in der Stechhilfe positioniert und gehalten werden. In einer bevorzugten Ausführungsform wird weiterhin durch die Formveränderung des Nadelkörpers der Schutzbereich des Nadelkörpers in seine erste Position überführt, so dass die Entsorgung einer gebrauchten Lanzette kein Verletzungsrisiko in sich birgt. Der Schutzbereich des Nadelkörpers und der Sperrmechanismus sind dann in einem Bauteil des Lanzettensystems realisiert.

Prinzipiell kann das Zusammenwirken der Halteelemente auf vielfältige Weise verhindert werden. Hierbei kann der Sperrmechanismus entweder direkt oder indirekt Einfluss auf die Halteelemente nehmen. Bei einem direkter Einfluss auf die Haltelemente wird vorteilhafterweise mindestens ein Halteelement in der Weise verändert, bedeckt oder zerstört, dass ein Zusammenwirken der Haltelemente nicht mehr möglich ist. Darüber hinaus sind auch Ausführungsformen denkbar, bei denen das Lanzettensystem aufgrund von magnetischen Eigenschaften des Systems innerhalb einer Stechhilfe positioniert wird. Eine Änderung der magnetischen Eigenschaften des Nadelkörpers kann somit ein erneutes Wiederverwenden des Lanzettensystems verhindern. Als Halteelemente des Systems sind dann die entsprechenden magnetischen Elemente des Nadelkörpers bzw. der Stechhilfe zu verstehen.

Da der Sperrmechanismus vorteilhafterweise ausschließlich ein wiederholtes Einführen des Lanzettensystems verhindert, jedoch kein Wiederverwenden einer bereits eingelegten Nadel, entspricht das Lanzettensystem auch den Anforderungen im Home-Monitoring-Bereich, wo das mehrfache Verwenden einer einmal eingelegten Nadel häufig gewünscht wird.

Die erfindungsgemäße Stechhilfe zur Blutentnahme besitzt eine Antriebseinheit mit einem Stößel, durch den eine Nadel aus einer Ruheposition in eine Stechposition bewegt wird. Im Stand der Technik sind eine Reihe von Antriebsmechaniken bekannt, die auf dem Gebiet der Blutentnahmegeräte eingesetzt werden können (z. B. US 5,314,442, WO 00/02482, US 3,030,959). Insbesondere werden in großem Umfang Antriebsmechaniken eingesetzt, die ihre Energie aus einer zuvor gespannten Feder beziehen. Im Rahmen der vorliegenden Erfindung werden vorzugsweise Antriebseinheiten eingesetzt, die eine geführte Bewegung des Stößels und der Nadel, beispielsweise aufgrund einer formschlüssigen Kopplung, ermöglichen, wie sie in dem Dokument DE 10053974 beschrieben wird. Weiterhin werden geführte Bewegungen der Nadel, zum Beispiel über Führungskulissen in EP 0 565 970 vorbeschrieben. Derartige Antriebsmechaniken sind aufgrund ihres geringen Einstichschmerzes bevorzugt. Jedoch ist das erfindungsgemäße System nicht auf eine bestimmte Antriebsmechanik beschränkt, sondern ist vielmehr mit vielfältigen möglichen Antriebseinheiten kombinierbar.

Ein wichtiger Aspekt der Erfindung stellt ein von der Antriebseinheit abnehmbares Lanzettensystem dar, in dem sich mindestens eine Nadel befindet, wobei das Lanzettensystem als disposible Einheit vorgesehen ist. Hierbei umfasst der Begriff Nadel sowohl eine klingenförmige, flachausgeprägte Stecheinheit sowie andere denkbare Ausführungsformen. Prinzipiell können im Rahmen der Erfindung Nadeln eingesetzt werden, wie sie grundsätzlich im Stand der Technik hinlänglich bekannt sind und in einem Lanzettensystem Anwendung finden. Im Stand der Technik wird dabei häufig die Kombination einer Nadel mit einem Grundkörper, der an die Stechhilfe ankoppeln kann, als Lanzette bezeichnet. Solche Lanzetten weisen oftmals einen Grundkörper aus Kunststoff auf, in dem eine Metallnadel angeordnet ist. Erfindungsgemäß ist es möglich, eine derartige Lanzette in das erfindungsgemäße Lanzettensystem zu integrieren. Es ist z. B. denkbar, dass der erfindungsgemäße Nadelkörper einen Grundkörper, wie er im Stand der Technik bei Lanzetten eingesetzt wird, enthält, wobei durch die Integration des Grundkörpers die erfindungsgemäße Funktionalität des Systems gewahrt bleibt. Der Nadelkörper ist dann entsprechend der beschriebenen Ausgestaltung mindestens zweiteilig ausgebildet. In einer bevorzugten Ausführungsform ist der Nadelkörper so ausgestaltet, dass eine Mehrzahl von Lanzetten in dem Nadelkörper angeordnet ist, so dass der Nadelkörper ein Magazin mit mehreren Lanzetten darstellt, wobei der jeweilige Grundkörper der Lanzette Teile des Nadelkörpers darstellen. In einer bevorzugten Ausführung wird folglich der Schutzbereich des Nadelkörpers durch das Magazingehäuse ausgebildet. Die Nadel und der Grundkörper sind innerhalb des Magazins, dann beweglich führbar. Vorzugsweise befinden sich die Nadeln innerhalb eines als Magazin ausgestalteten erfindungsgemäßen Nadelkörpers in voneinander separierten Kammern, um eine Kontamination ungebrauchter Nadeln durch bereits verwendete Nadeln bei einer Remagazinierung zu verhindern.

Zum Ausführen eines Stechvorgangs sind Bereiche des Nadelkörpers vorteilhafterweise analog zu der in DE 10053974 bereits dargestellten Systems ausgestaltet, so dass die einzelnen Nadeln des Systems mit der Antriebseinheit der Stechhilfe aktiv koppeln können. Ausführungsformen, die ebenfalls zum Antreiben von Nadeln innerhalb eines Magazins einer Stechhilfe dienen können, sind z. B. in den Dokumenten DE 10053974,

US 4,990,154 und US 5,074,872 beschrieben. Die nebeneinander angeordneten Kammern, in denen sich die Lanzetten einzeln befinden, werden zum Ausführen eines Stechvorgangs nacheinander relativ zur Antriebseinheit so positioniert, dass jeweils eine einzelne Nadeln an den Stößel der Antriebseinheit angekoppelt werden kann. Besonders vorteilhaft erweisen sich auch hier Magazine in Form einer Trommel mit parallel zur Längsachse der Trommel angeordneten Kammern, in denen sich die Nadeln befinden.

Das Lanzettensystem umfasst weiterhin vorteilhafterweise einen Nadelkörper, der die Nadelspitze mit dem Schutzbereich des Körpers zumindest teilweise umgibt, wenn die Nadel sich in einer Ruheposition befindet. Zum Ausführen des Stechvorgangs findet eine räumliche Trennung des Schutzbereiches des Nadelkörpers von der Nadelspitze statt, so dass der Schutzbereich des Nadelkörpers während des Stechvorgangs zu keinen Behinderungen führt. Beim Auswurf des Lanzettensystems aus der Stechhilfe bleibt die Ruheposition der Nadel erhalten, so dass ein Schutz vor der ausgeworfenen Nadelspitze gegeben ist, wobei zusätzlich durch den erfindungsgemäßen Sperrmechanismus ein Wiederverwenden des Lanzettensystems verhindert wird. Es ist jedoch auch möglich, dass erst beim Auswurf des Lanzettensystems der Schutzbereich des Nadelkörpers in seine erste Position überführt wird, so dass ein Schutz der Nadelspitze erst aufgrund des Auswurfes umgesetzt wird. In einer bevorzugten Ausführungsform befindet sich die Nadel im ungebrauchten Zustand auch vor dem Einführen in die Stechhilfe in einer Ruheposition, so dass sowohl beim Einführen der Nadel, als auch nach dem Auswurf eine Verletzung an der Nadelspitze vermieden wird und es zu keiner Kontamination kommen kann.

Unabhängig vom Schutz der Nadelspitze, kann ein erfindungsgemäßer Sperrmechanismus z. B. beim Auswurf bzw. Einsetzen des Lanzettensystems aus oder in die Stechhilfe betätigt werden. Prinzipiell kann der Sperrmechanismus oder der Schutz der Nadelspitzen, aber auch separat oder durch einzelne Bedienungsschritte der Stechhilfe, z. B. beim Stechvorgang selbst, aktiviert werden. Generell sind alle möglichen Kombinationen denkbar, inwieweit der Sperrmechanismus und der Schutz der Nadelspitze gleichzeitig oder hintereinander gewährleistet werden.

Die Ausgestaltung des Sperrmechanismus kann vielfältig sein, wobei die Form des Nadelkörpers in der Weise verändert wird, dass ein Wiedereinführen des einmal ausgeworfenen Lanzettensystems nicht mehr möglich ist und der Sperrmechanismus eine Verschiebung von mindestens einem Teil des Nadelkörpers bewirkt, welches so mit der Stechhilfe zusammenwirkt, dass eine Veränderung seiner Position ein Wiedereinführen des Lanzettensystems blockiert. Dies ist z. B. dann gegeben, wenn der Sperrmechanismus eine Ausnehmung im Nadelkörper, die ein Halteelement ausbildet, verschließt, oder eine Ausnehmung im Nadelkörper generiert wird, die für ein Zusammenwirken des Lanzettensystems mit einer Stechhilfe wesentlich ist.

Erfindungsgemäß wird die Aktivierung des Sperrmechanismus sowie die erste Position des Schutzbereiches, in der der Schutzbereich die Nadelspitze zumindest teilweise umgibt, durch ein Zusammenwirken zwischen Lanzettensystem und Stechhilfe erzielt.

Eine wichtige Anforderung an das Lanzettensystem liegt in der Sterilität der Nadelspitze, die zur Erzeugung einer Wunde in einem dafür vorgesehenen Körperteil vorgesehen ist. Die Sterilität der Nadelspitze muss dabei über einen langen Zeitraum gewährleistet sein, der sich von der Produktion des Lanzettensystems bis zu seinem Gebrauch erstreckt. Bei der Herstellung des Lanzettensystems kann eine Sterilität, wie im Stand der Technik üblich, z. B. durch Gamma-Strahlung erzielt werden. Zum Aufrechterhalten der Sterilbedingung kann das Lanzettensystem in eine Umverpackung, beispielsweise einen Polyethylenbeutel, eingeschweißt werden. In einer anderen Ausführungsform kann beispielsweise die Öffnung des Lanzettensystems zum Austritt der Nadelspitze aus dem Schutzbereich des Nadelkörpers durch Siegelfolie verschlossen werden. Diese können vorzugsweise abziehbare Siegelfolien sein, die der Benutzer vor dem Gebrauch des Lanzettensystems entfernt. Es können jedoch auch dünne Folien verwendet werden, die erst bei der Benutzung der Nadel von der Nadelspitze durchstoßen werden, so dass dem Benutzer zusätzliche Handhabungsschritte erspart bleiben. Solche Folien können bereits integral im Herstellungsprozess des Lanzettensystems, in der Regel mittels eines Spritzgussprozesses, eingesetzt werden.

Des Weiteren wird im Stand der Technik in der Anmeldung WO 01/66010 ein Sterilschutz durch ein Elastomer beschrieben, das die Nadelspitze einschließt und sie somit vor Kontamination schützt. Dieser Sterilschutz kann entweder während des Stechvorgangs durchstochen werden oder vor Gebrauch von dem Benutzer entfernt werden.

In einer vorteilhaften Ausführungsform kann der Schutzbereich des Nadelkörpers einen Sterilschutz beinhalten und/oder durch diese im Wesentlichen gebildet werden. Hierbei dient z. B. das Elastomer des Sterilschutzes selbst als Schutzbereich des Nadelkörpers, in dem die Nadelspitze relativ zu dem Elastomer beweglich geführt werden kann. Ein weiterer Teil des Nadelkörpers, der unabhängig vom Sterilschutz betätigt werden kann, ermöglicht eine Veränderung des Nadelkörpers und stellt den Sperrmechanismus dar. Dies setzt jedoch voraus, dass der Sterilschutz reversibel die Nadelspitze freigibt bzw. wieder umschließen kann, wie es zum Beispiel im Falle eines Elastomerenschutzes (WO 01/66010) gegeben ist, bei dem das Elastomer während des Stechvorgangs zunächst durchstochen und anschließend die Nadelspitze wieder in das Elastomer zurückgezogen wird. Im aufgeführten Beispiel ändert folglich die Nadelspitze ihre Position relativ zum Sterilschutz während des Stechvorgangs, wobei die Nadelspitze nach dem Stechvorgang in ihrer Ruheposition vom Sterilschutz geschützt wird. Prinzipiell sind viele mögliche Ausführungsformen eines Sterilschutzes denkbar, so dass das erfindungsgemäße System auf keine spezielle Ausführungsform eines Sterilschutzes beschränkt ist.

Nachfolgend wird anhand der Figuren das erfindungsgemäße System beispielhaft erläutert, ohne dass hierdurch eine Einschränkung auf die einzelnen Beispiele gegeben ist.
- Figur 1:: Zweiteiliges Lanzettensystem
- Figur 2:: Lanzettensystem, bei dem der Sperrmechanismus während des Stechvorgangs aktiviert wird
- Figur 3:: Lanzettensystem mit einem Sperrmechanismus, der beim Systemauswurf aktiviert wird
- Figur 4:: Lanzettensystem mit einem Sperrmechanismus, der ein Ankoppeln einer Stechhilfe an das Lanzettensystem blockiert
- Figur 5:: Lanzettensystem mit einem Sperrmechanismus, der beim Einlegen in die Stechhilfe aktiviert wird
- Figur 6:: Stechhilfe mit Lanzettenmagazin
- Figur 7:: Lanzettensystem mit einem Sperrmechanismus, der zur Verbreiterung des Nadelkörpers führt

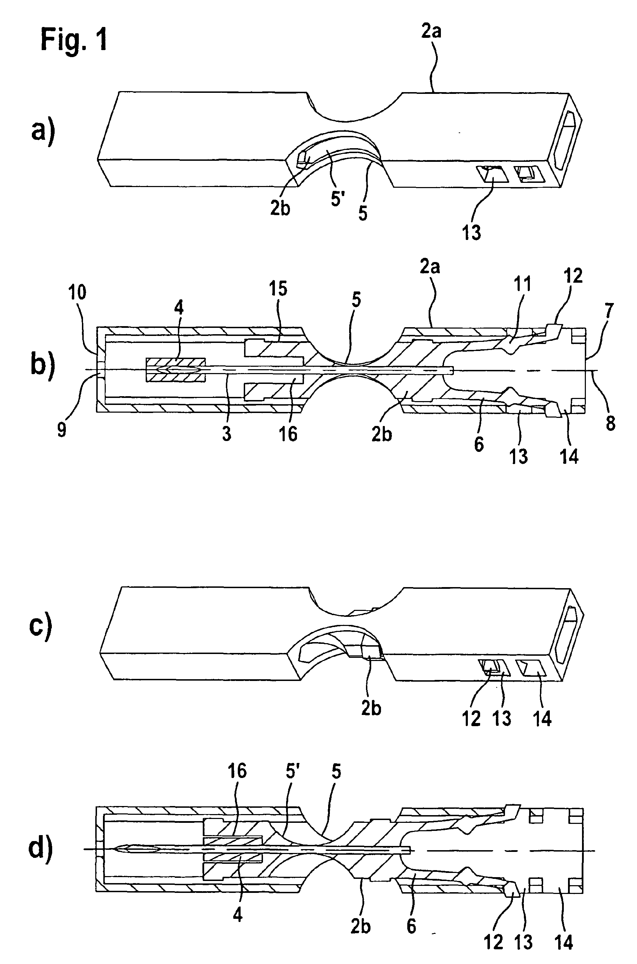

Figur 1 zeigt ein Lanzettensystem (1), das im Wesentlichen zweiteilig ist. Hierbei zeigen die Figuren 1b und 1d jeweils einen Querschnitt, durch das in der Figur 1a und 1c gezeigten Lanzettensystem vor bzw. nach dem Gebrauch. Das System verfügt über eine Nadel (3), die an der vorderen Nadelspitze mit einem elastomeren Schutz (4) steril verpackt ist. Derartige Elastomere, die die Sterilität der Nadelspitze gewährleisten, sind z. B. aus dem Dokument WO 01/66010 bekannt, auf das an dieser Stelle Bezug genommen wird. Die metallische Nadel (3) ist an einem Kunststoffkörper (2b) befestigt und mit diesem fest verbunden. Der Kunststoffkörper verfügt über einen hinteren Bereich (6), der zur Ankopplung des Lanzettensystems an einen Antriebsstößel vorgesehen ist, so dass die Nadel entlang der Achse (8) in Einstichrichtung bewegt werden kann. Der hintere Bereich (6) des Kunststoffkörpers, der einen Teil des Nadelkörpers darstellt, wird durch zwei Arme gebildet, die mittels der Vorsprünge (11) mit einem Antriebsstößel einer Stechhilfe (nicht gezeigt) eine formschlüssige Verbindung beim Stechvorgang eingehen können. Eine formschlüssige Verbindung zwischen Antriebsstößel und Lanzette ist beispielsweise in dem Dokument DE 10053974 beschrieben, auf das an dieser Stelle ebenfalls Bezug genommen wird. Es ist natürlich auch jeder andere mögliche Ankopplungsmechanismus zur Ausführung des Stechvorgangs, wie im Stand der Technik weiterhin bekannt ist, denkbar. Die Nadel (3) und der mit ihr fest verbundene Kunststoffkörper (2b) sind beweglich im Kunststoffkörper (2a) gelagert, der den Schutzbereich des Nadelkörpers darstellt. Innerhalb dieses Nadelkörpers ist die Nadel und der zweite Teil des Nadelkörpers (2b) entlang der Einstichrichtung verschiebbar. Der Schutzbereich des Nadelkörpers (2a) verfügt über eine untere Wand (10), die ein Loch (9) aufweist, aus der die Nadelspitze beim Stechvorgang austreten kann. Des Weiteren weist der Nadelkörper an seinem oberen Ende eine Öffnung (7) auf, durch die der Antriebsstößel einer Stechhilfe in den Nadelkörper einfahren kann, um mit dem zweiten Teil des Nadelkörpers eine formschlüssige Verbindung einzugehen und den Stechvorgang durchzuführen. Der Schutzbereich des Nadelkörpers (2a) verfügt weiterhin über zwei Ausnehmungen (13 und 14), die ein Einrasten in den zweiten Teil des Nadelkörpers (2b) erlauben. Um ein Einrasten des zweiten Teils des Nadelkörpers in den Schutzbereich zu ermöglichen, verfügt der hintere Bereich (6) des zweiten Teils des Nadelkörpers weiterhin über Rastnasen (12), die in einer ersten Ruheposition des Lanzettensystems vor dessen Gebrauch in die Ausnehmungen (14) eingreifen und dort aufgrund des gespreizten Zustandes der Arme (6) des zweiten Teils des Nadelkörpers gehalten werden. Sowohl der Schutzbereich als auch der zweite Teil des Nadelkörpers verfügen in ihrem mittleren Bereich über muldenförmig ausgeprägte Verjüngungen (5 und 5') der Körper, so dass im ersten Ruhezustand vor Gebrauch des Lanzettensystems die Verjüngungen (5 und 5') passgenau ineinander liegen, und das Lanzettensystem an dieser Stelle entsprechend Figur 1a ausgebildet ist.

Zur Ausführung des Stechvorgangs greift ein Stößel (nicht gezeigt) der Stechhilfe durch die Öffnung (7) in das Lanzettensystem ein, wobei der Stößel eine formschlüssige Verbindung mit den Armen (6) des zweiten Teils des Nadelkörpers eingeht. Hierdurch bedingt werden die Arme (6) zusammengedrückt, so dass die Nasen (12) der Arme (6) nicht mehr länger in die Ausnehmungen (14) eingreifen und die Nadel entlang der Einstichrichtung (8) vorwärts bewegt werden kann. Dabei wird der Elastomerenschutz (4) zunächst gegen die untere Wand (10) des Nadelkörpers (2a) gedrückt. Wird der Stechvorgang weiter durchgeführt, wird die Nadel durch den Elastomerenschutz hindurch angetrieben, so dass sie aus der Öffnung (9) der unteren Wand (10) austreten kann und in einem dafür vorgesehenen entsprechenden Körperteil eine Wunde erzeugt. Der Elastomerenschutz (4) wird währenddessen von der Wand (10) zurückgehalten, wobei sich der vordere Teil (15) des zweiten Teils des Nadelkörpers (2b) entsprechend der Ausnehmung (16) über den Elastomerenschutz stülpen kann. Nach der Ausführung des Stechvorgangs wird anschließend der Nadelkörper (2b) mit der Nadel aufgrund der formschlüssigen Kopplung mit dem Antriebsstößel in den Schutzbereich (2a) zurückgezogen. Gelangen die hinteren Arme (6) in den hinteren Schutzbereich des Nadelkörpers (2a), so können die Nasen (12) beim Zurückziehen des Nadelkörpers (2b) in die Ausnehmung (13) des Nadelkörpers (2a) einrasten. Das Lanzettensystem befindet sich nun in einer zweiten Ruheposition nach dem Stechvorgang. In dieser Position ragt der zweite Teil des Nadelkörpers (2b) aus der Öffnung (5) des Schutzbereiches des Nadelkörpers (2a) in der Weise hervor, dass die Nadelkörperform in diesem Bereich eine Veränderung erfährt. Die Verjüngungen (5 und 5') liegen nicht mehr passgenau ineinander. In einer entsprechend ausgestalteten Stechhilfe, die nur das Einführen des Lanzettensystems zulässt, wenn die Verjüngung (5) des Nadelkörpers entsprechend Figur 1a vollständig ausgebildet ist, wird das Einführen eines bereits gebrauchten Lanzettensystems entsprechend Figur 1c blockiert.

In dem in Figur 1 gezeigten Beispiel wird sowohl der Sperrmechanismus als auch eine Überführung des Schutzbereiches des Nadelkörpers in eine erste Position, während des Stechvorgangs realisiert. Wird das Lanzettensystems nach Gebrauch der Nadeln ausgeworfen, ist die Form des Systems bereits in der Weise verändert worden, dass ein Wiedereinführen des Lanzettensystems in eine entsprechend ausgestaltete Stechhilfe nicht mehr möglich ist. Des Weiteren wird die Nadelspitze vollständig von dem Schutzbereich des Nadelkörpers umgeben, so dass keine Verletzungsgefahr für Dritte, z. B. bei der Müllbeseitigung, gegeben ist.

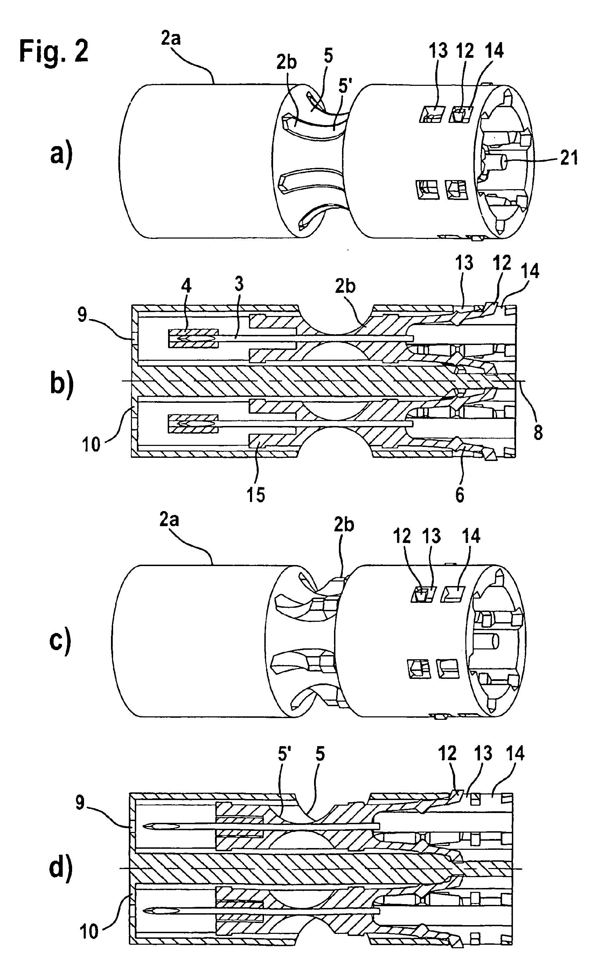

Figur 2 zeigt ein Lanzettensystem in Form eines im Wesentlichen rund ausgebildeten Lanzettenmagazins. Im Vergleich zu Figur 1 wurde der Schutzbereich des Nadelkörpers (2a) lediglich in Form eines Magazins ausgebildet, so dass eine Mehrzahl von Nadeln (3) in ihm beweglich geführt werden kann.

Figur 2a zeigt die äußere Ansicht eines Magazins. Das Magazingehäuse, das den Schutzbereich des Nadelkörpers ausbildet, ist ähnlich zu Figur 1 ausgestaltet und verfügt über Ausnehmungen (13 und 14), in die jeweils die Rastnasen (12) des jeweils zweiten Teils des Nadelkörpers (2b) eingreifen können. Das Lanzettensystem verfügt über eine konzentrisch im Schutzbereich des Nadelkörpers angeordnete Magazinachse (21), durch die das Lanzettensystem in einer Stechhilfe gelagert wird. Um die Achse (21) ist eine Rotation des Lanzettensystems möglich, so dass jeweils eine Nadel relativ zu einem in der Stechhilfe verwendeten Antriebseinheit (nicht gezeigt) positioniert werden kann. Analog zu dem in Figur 1 dargestellten Lanzettensystem verfügt das Lanzettensystem in Figur 2 ebenfalls über Verjüngungen (5) innerhalb des Schutzbereiches des Nadelkörpers (2a), die in Form von Öffnungen ausgebildet sind, wobei die Öffnungen ebenfalls durch den zweiten Teil des Nadelkörpers (2b) im Wesentlichen passgenau geschlossen werden.

Figur 2b zeigt einen Querschnitt durch das in Figur 2a dargestellten Lanzettenmagazin. Analog zu Figur 1 erkennt man ein ähnlich aufgebautes System, das lediglich im Vergleich zu Figur 1 aus einer Mehrzahl von Nadeln besteht, die mit einem entsprechend zugeordneten zweiten Teilen des Nadelkörpers (2b) ausgestattet sind. Das in Figur 2 dargestellte Lanzettensystem ist folglich mehrteilig, wobei es über einen äußeren Schutzbereich (2a) des Nadelkörpers und mehreren zweiten Teilen (2b) des Nadelkörpers verfügt. Figur 2c und 2d stellen das Lanzettensystem nach dem Gebrauch dar, wobei alle Nadeln des Lanzettensystems bereits zum Einstich verwendet wurden. Es ist natürlich auch denkbar, dass in dem Lanzettensystem nur ein Teil der Nadeln bereits verwendet wurde. In diesem Fall würden nur durch einen Teil der Öffnungen (5) des Schutzbereiches des Nadelkörpers (2a) jeweils ein zweiter Teil (2b) der Nadelkörper ragen, während ein anderer Teil der Öffnungen, wie in Figur 2a dargestellt, passgenau durch die Nadelkörper (2b) verschlossen werden. In Abhängigkeit von dem Zusammenwirken des Lanzettensystems und der Stechhilfe sind Ausführungsformen denkbar, die das Wiedereinführen des Lanzettensystems in eine Stechhilfe bereits blockieren, sobald ein Teil der Nadeln benutzt wurde oder erst dann eine Blockierung erfolgt, nachdem vollständig alle Nadeln im Lanzettensystem verwendet wurden. Vorteilhafterweise ist es auch denkbar, dass das Wiedereinführen eines zum Teil benutzten Lanzettensystems in die Stechhilfe erst dann ermöglicht wird, wenn das System relativ zum Antriebsstößel in der Weise positioniert wird, dass nur ungebrauchte Lanzetten vom System verwendet werden können.

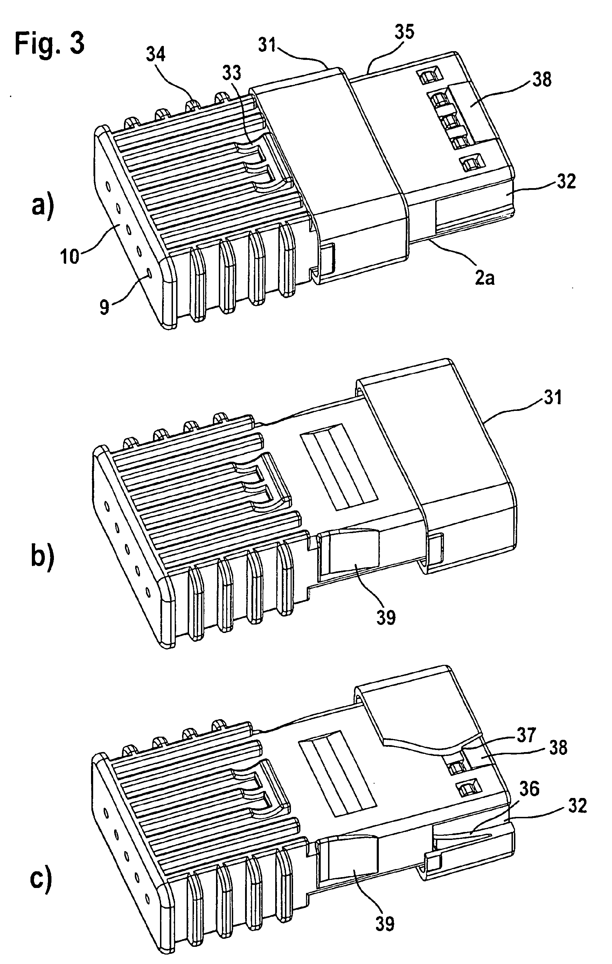

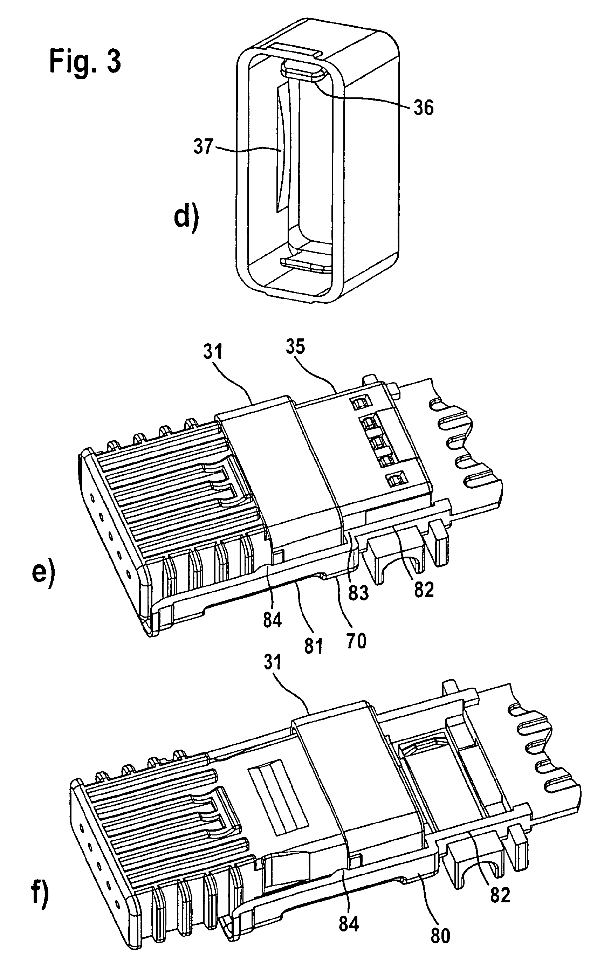

Figur 3 zeigt einen rechteckig ausgebildeten Nadelkörper, der ebenfalls in Form eines Magazins eine Mehrzahl von Nadeln beinhaltet. Der Schutzbereich des Nadelkörpers (2a) enthält ebenfalls an einem unteren Ende (10) Öffnungen (9), aus denen die Nadeln zum Ausführen eines Stechvorgangs austreten können. Während der Ruheposition, d. h. wenn kein Stechvorgang ausgeführt wird, befinden sich die Nadelspitzen der Nadeln (nicht gezeigt) innerhalb des Schutzbereiches des Nadelkörpers (2a), in dem die Nadeln beweglich geführt werden können. Der Nadelkörper (2a) enthält in einem unteren Bereich (34), der am unteren Ende (10) des Nadelkörpers angrenzt, Rillen, die eine bessere Griffigkeit und damit eine erleichterte Handhabung für den Benutzer gewährleisten sollen. In diesem Bereich (34) sind Vertiefungen (33) als Halteelemente vorgesehen, die in einer entsprechend ausgestalteten Stechhilfe ein Einrasten des Lanzettensystems in die Stechhilfe beim Einführen bewirken. In der Mitte des Nadelkörpers (2a) befindet sich der Sperrmechanismus (31) als Teil des Nadelkörpers (2a), der beweglich zu einem oberen Teil (35) des Nadelkörpers (2a) geführt werden kann, und zunächst in seiner Ausgangsposition durch federnd gelagerte Arme (39) gehalten wird. Am oberen Teil (35) ist weiterhin eine Vertiefung (32) angebracht, die eine Einrasten des Sperrmechanismus (31) bewirkt, wenn der Sperrmechanismus (31) entlang des oberen Nadelkörperteils (35) geführt wird.

Figur 3b zeigt das Lanzettensystem nach dessen Gebrauch. Wie Figur 3b veranschaulicht, ist der Sperrmechanismus (31), der in Form eines Ringes den Nadelkörper (2a) umgibt, nun am oberen Ende des Nadelkörpers positioniert, sodass der Sperrmechanismus (31) in dieser Position eine Verbreiterung des Nadelkörperbereiches (35) bewirkt. Ist der Sperrmechanismus (31) einmal in seiner Position eingerastet, ist ein anschließendes Wiedereinführen des Lanzettensystems aufgrund des vergrößerten Nadelkörpers nicht mehr möglich.

Figur 3c und d veranschaulichen näher die Funktionsweise des Sperrmechanismus (31), der in dem oben dargestellten Lanzettensystem Anwendung findet. Zum Einrasten des Sperrmechanismus (31) mit dem oberen Bereich (35) verfügt der Sperrmechanismus über Rastarme (36), die in die Vertiefungen (32) eingreifen. Zur Sicherung gegen ein Wiederverschieben des Sperrmechanismus (31), in der Figur 3b und c dargestellten Position, werden die Rastarme (36) federnd gegen die untere Kante der Vertiefung (32) gelagert. Gleichzeitig dient der Anschlag (37) als zusätzliches Widerlager des Sperrmechanismus (31) gegen den Vorsprung (38) am oberen Bereich des Nadelkörperteils (35). Wird das Magazin, wie in Figur 3e dargestellt, zum erstmaligen Gebrauch in ein Stechhilfegehäuse (70) eingeführt, wird der hintere Bereich (35) des Magazingehäuses in einer entsprechend verjüngten Stelle (82) des Gehäuses (70) positioniert. Der vordere Bereich (80) des Gehäuses (70) ist hingegen verbreitet ausgebildet, sodass der verbreitete Durchmesser des Lanzettensystems aufgrund des als Sperrmechanismus ausgebildeten Ringes entsprechend in das Stechhilfengehäuse eingelegt werden kann. In dieser Position wird das Lanzettensystem derartig in der Stechhilfe gehalten, dass eine Antriebseinheit (nicht gezeigt) der Stechhilfe in das Magazingehäuse eingreifen kann, um an eine Nadel des Lanzettensystems anzukoppeln. Das Stechhilfengehäuse verfügt weiterhin über zwei Anschläge (83, 84), an die der Sperrmechanismus (31) in dieser Position des Lanzettensystems in der Stechhilfe angrenzt. Wird das Lanzettensystem nach dessen Gebrauch aus dem Stechhilfegehäuse entnommen, bewirkt der Anschlag (84) zunächst, dass der Sperrmechanismus (31) ortsfest im Stechhilfengehäuse verbleibt, während das Magazingehäuse aus dem Bereich (82) der Stechhilfe hinausgezogen wird. Hierdurch wird der Sperrmechanismus (31) entlang des Nadelkörpers hin zum oberen Bereich (35) des Nadelkörpers verschoben. Dabei findet eine Einrasten des Sperrmechanismus (31) mit dem Nadelkörper statt, wobei der Vorsprung (38) und der Anschlag (37) ein weiteres Verschieben des Sperrmechanismus entlang des Nadelkörpers blockiert. Wird der Sperrmechanismus (31) gegen den Vorsprung (38) gelagert, führt eine weitere Zugbewegung am Magazingehäuse dazu, dass der Widerstand des Anschlages (84) überwunden wird und das Magazin aus der Stechhilfe entnommen werden kann. Das Magazin liegt nun außerhalb des Gehäuses in einem gebrauchten Zustand, wie in Figur 3b gezeigt, vor, wobei der Sperrmechanismus (31) aufgrund der Rasthaken (36) sowie des Anschlages (37) fest am Nadelkörper positioniert ist. Beim erneuten Einschieben des Lanzettenmagazins in das Stechhilfegehäuse, kann das Magazin nun nicht mehr aufgrund des verbreiteten Umfangs des oberen Bereiches (35) des Nadelkörpers in den verjüngten Bereich (82) der Stechhilfe hineingeschoben werden. Eine Positionierung des Lanzettensystems an seinen ursprünglichen Ort in der Stechhilfe ist somit nicht mehr möglich. Das Lanzettensystem kann nicht mehr in die Stechhilfe gehalten werden. Eine Ankopplung einzelner Nadeln an die Antriebseinheit der Stechhilfe zur Ausführung eines Stechvorgangs wird verhindert. Nach dem Auswurf des Lanzettensystems kann darüber hinaus der Benutzer anhand des verschobenen Ringes leicht optisch erkennen, dass es sich bei dem Lanzettensystem um ein bereits gebrauchtes System handelt. Zu diesem Zwecke ist es auch denkbar, dass der Sperrmechanismus (31) farblich hervorgehoben wird.

Alternativ zu der beschriebenen Veränderung des Nadelkörpers (2a) ist es z. B. auch denkbar, dass eine Verschiebung des Sperrmechanismus (31) über die Vertiefungen (33) des Lanzettensystems geführt werden kann. In diesem Falle würde ein Wiedereinführen des Lanzettensystems in eine Stechhilfe dadurch verhindert werden, dass ein Einrasten des Lanzettensystems in die Stechhilfe nicht mehr möglich wäre. Weitere Ausführungsformen mittels eines verschiebbar gelagerten Sperrmechanismus sind denkbar, die zum Beispiel zu einer Verkleinerung des Nadelkörpers im oberen Bereich (35) führen. Hierbei würde ein ungebrauchtes Lanzettensystem, z. B. ausgehend von dem Zustand, wie in Figur 3b dargestellt, zunächst in eine Stechhilfe eingelegt. Ein bereits benutztes Lanzettensystem wäre dann dadurch gekennzeichnet, dass der Sperrmechanismus (31) in der Weise über den Nadelkörperbereich (35) geschoben würde, so dass es zu einer Verkleinerung des Nadelkörpers (2a) im oberen Bereich (35) käme. Figur 3a würde somit den ausgeworfenen Zustand des Systems darstellen. Eine entsprechend ausgestaltete Stechhilfe würde dann zum Beispiel über Halteelemente verfügen, die mit einem auf diese Weise veränderten Lanzettensystem nicht mehr wechselwirken könnten und ein erneutes Einführen in das Lanzettensystem nicht mehr möglich ist. Die Rastelemente des Sperrmechanismus bzw. des Nadelkörpers (2a) müssten dann entsprechend angepasst werden. Des Weiteren ist es auch denkbar, dass mittels eines verschiebbar angebrachten Sperrmechanismus (31) sowohl das Wiedereinführen des Lanzettensystems blockiert wird, als auch gleichzeitig ein Schutz der Nadelspitzen gewährleistet wird. In diesem Fall würden die Nadelspitzen nach dem Stechvorgang nicht, wie in Fig. 3 dargestellt, in einen Schutzbereich des Nadelkörpers zurückgezogen. Ein Schutz der Nadelspitzen in einer Ruheposition wäre folglich nicht automatisch gegeben. Beispielsweise wird der Schutz vor den Nadelspitzen dann erst beim Auswurf des Lanzettensystems aus der Stechhilfe gewährleistet. Gemäß dem in Figur 3 a dargestellten Sperrmechanismus führt eine Verschiebung des Sperrmechanismus (31) dann zur Verlängerung des Nadelkörpers in dem Bereich der Nadelspitzen, sodass die Nadelspitzen schützend vom Sperrmechanismus umgeben werden, wobei gleichzeitig der Sperrmechanismus aufgrund einer Formveränderung des Körpers wirkt. In diesem Fall dient ein Teil des Nadelkörpers sowohl als Sperrmechanismus, als auch gleichzeitig als Schutzbereich des Nadelkörpers, der den Bereich der Nadelspitze im ausgeworfenen Zustand des Lanzettensystems umgibt. Der Schutzbereich des Nadelkörpers und der Sperrmechanismus sind dann in einem Bauelement des Nadelkörpers verwirklicht.

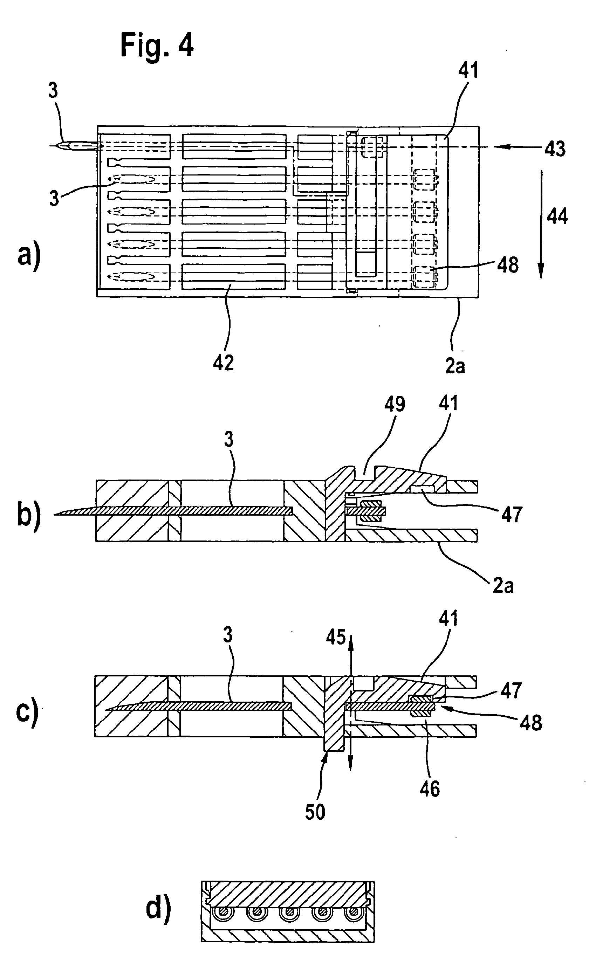

Figur 4 zeigt ein rechteckiges aufgebildetes Lanzettensystem, in dem mehrere Nadeln in Kammern (42) des Schutzbereiches des Nadelkörpers (2a) positioniert sind. Der Schutzbereich des Nadelkörpers verfügt in seinem oberen Bereich über ein Sperrmechanismus (41) in Form einer Taste, die oberhalb des Schutzbereiches des Nadelkörpers angeordnet ist und entlang der Richtung (45) beweglich zum Schutzbereich des Nadelkörpers verschiebbar ist. Die Taste verfügt in ihren oberen Bereich über eine Führungsnut (49), in die eine entsprechend ausgestaltete Lippe der Stechhilfe (nicht gezeigt) eingreift, sodass eine sichere Positionierung des Lanzettensystems in der Stechhilfe gewährleistet wird. Auf diese Weise positioniert, kann ein Antriebstößel der Stechhilfe (nicht gezeigt) an den hinteren Bereich (48) der Nadel (3) ankoppeln, sodass ein Stechvorgang ausgeführt werden kann. Die Nadel wird hierfür entlang der Richtung (43) relativ zum Schutzbereich des Nadelkörpers bewegt. Dabei wird die Nadelspitze vom Schutzbereich (2a) des Nadelkörpers freigegeben. Analog zu den bereits dargestellten Systemen erfolgt nach dem Stechvorgang eine Remagazinierung der Nadel und die Nadelspitze wird innerhalb des Nadelkörpers (2a) zurückgezogen. Zum Weitertakten des Magazins wird der Antriebstößel der Stecheinheit entlang der Richtung (44) bewegt, bis der Stößel mit einer in der nebenliegenden Kammer (42) positionierten Nadel ankoppeln kann, um einen erneuten Stechvorgang auszuführen. Soll das Lanzettensystem in der Stechhilfe ausgewechselt werden, muss zu diesem Zweck zunächst der Antriebsstößel außerhalb des hinteren Bereiches (46) des Nadelkörpers (2a) geführt werden. Hierfür wird das Magazin erneut weitergetaktet, wobei gleichzeitig die Taste (41) durch eine gehäuseseitige Rampe der Stechhilfe niedergedrückt wird. Die Taste (41) ist nun wie in Figur 4c und d dargestellt innerhalb des Lanzettensystems verschoben, sodass der Bereich (50) der Taste aus dem Boden des Nadelkörpers (2a) hervorragt. In dieser Position, umgreift ein Ausnehmung (47) der Taste (41) den hinteren Bereich (48) der Nadel (3). Auf diese Weise kann, wie durch die Frontansicht der Figur 4d veranschaulicht, ein erneutes Ankoppeln der Stechhilfe an das Lanzettensystem blockiert werden. Das Ausführen eines Stechvorgangs mit einem Lanzettensystem gemäß Figur 4c oder d wird folglich verhindert. Des weiteren wird ein Wiedereinführen des Lanzettensystems in die Stechhilfe aufgrund der Formveränderung des Nadelkörpers im Bereich (50) blockiert. Eine Positionierung des Lanzettensystems über die Führungsnut (49) als Teil eines Halteelementes unterbleibt folglich.

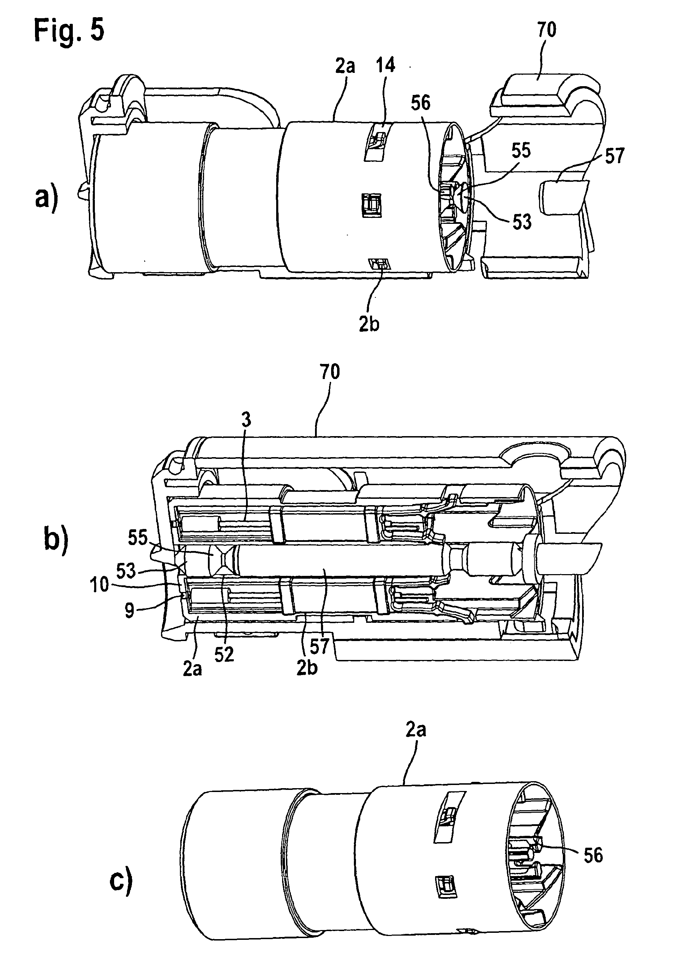

Figur 5 zeigt ein rund geformtes Lanzettensystem, in dem ebenfalls mehrere Nadeln innerhalb des Nadelkörpers angeordnet sind. Analog zu Figur 2 verfügt das Lanzettensystem über einen mehrteiligen Nadelkörper. Entlang der Rotationsachse des Lanzettensystems ist ein Kanal (52) angeordnet, in dem sich ein Stöpsel (53) am oberen Ende des Lanzettensystems befindet. Der Stöpsel (53) wird von spreizbaren Haltearmen (56) in seiner ersten Position gehalten, in der das Lanzettensystem im ungebrauchtem Zustand vorliegt. Die Haltearme (56) greifen hierbei in eine Verjüngung des Stöpsels (53) ein, die durch schräg aufeinander zulaufende Ebenen (55) des Stöpsels gebildet wird. Beim Einführen des Lanzettensystems in eine Stechhilfe (70) wird mittels eines Zentrierstößels (57) der Stechhilfe der Stöpsel (53) innerhalb des Kanals (52) in Richtung der Nadelspitzen gedrückt. Aufgrund der schräg ausgebildeten Ebenen (55) des Stöpsels (53) werden die Haltearme (56) beim Eindrücken des Stöpsels gespreizt. In dem gespreizten Zustand ist es dem Stößel (57) möglich, zwischen die Haltearme (56) des Lanzettensystems eingreifen zu können. Der Stößel (57) kann somit nahezu vollständig in das Lanzettensystem eingebracht werden und dient hier zur Lagerung und Positionierung des Magazins. Eine entsprechend ausgestaltete Antriebseinheit der Stechhilfe kann somit relativ zu den Lanzetten des Systems orientiert werden, sodass ein Ankoppeln an eine Lanzette möglich ist und ein Stechvorgang ausgeführt werden kann. Nach dem Gebrauch des Magazins wird dieses wieder aus der Stechhilfe entfernt. Hierbei wird der Stößel (57) aus dem Inneren des Magazingehäuses hinausgezogen, wobei der Stöpsel (53) am unteren Ende des Magazins im Bereich der Nadelspitzen verbleibt. Ein einmal verwendetes Lanzettensystem ist folglich, wie in Figur 5c dargestellt, ausgestaltet, wobei der Stöpsel (53) nicht mehr im oberen Bereich der Haltearme (56) gehalten wird. Bei einem erneuten Versuch, das bereits gebrauchte Lanzettensystem in die Stechhilfe einzuführen, trifft der Stößel (57) direkt auf den oberen Bereich (56) der Haltearme auf, die im nicht gespreizten Zustand ein Eindringen des Stößels in das Lanzettensystem verhindern. Aufgrund des fehlenden Stöpsels (53) ist es dem Stößel (57) der Stechhilfe nicht möglich, die Haltearme zu spreizen. Ein Einlegen des Lanzettensystems in die Stechhilfe wird auf diese Weise verhindert.

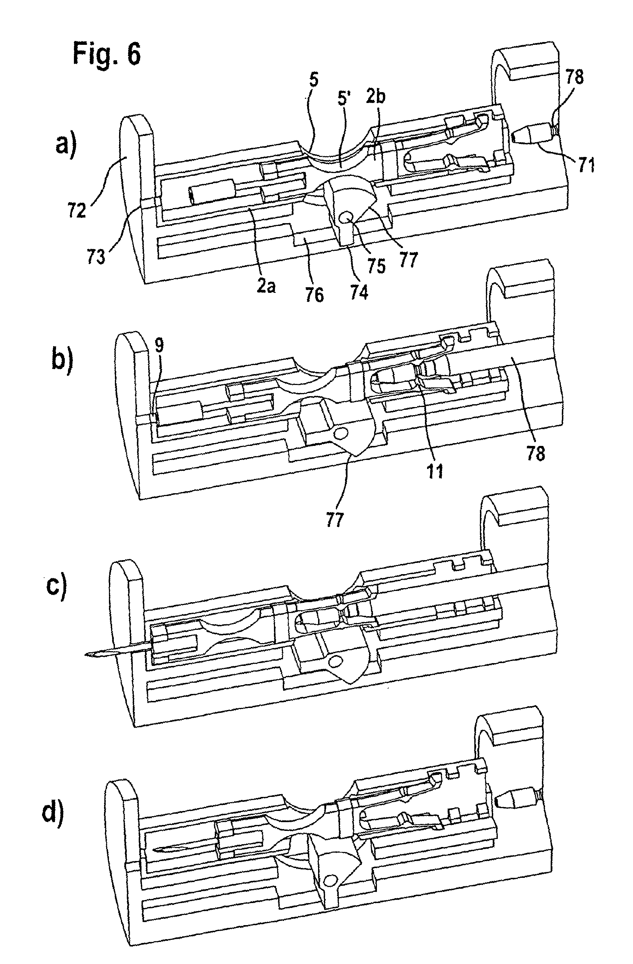

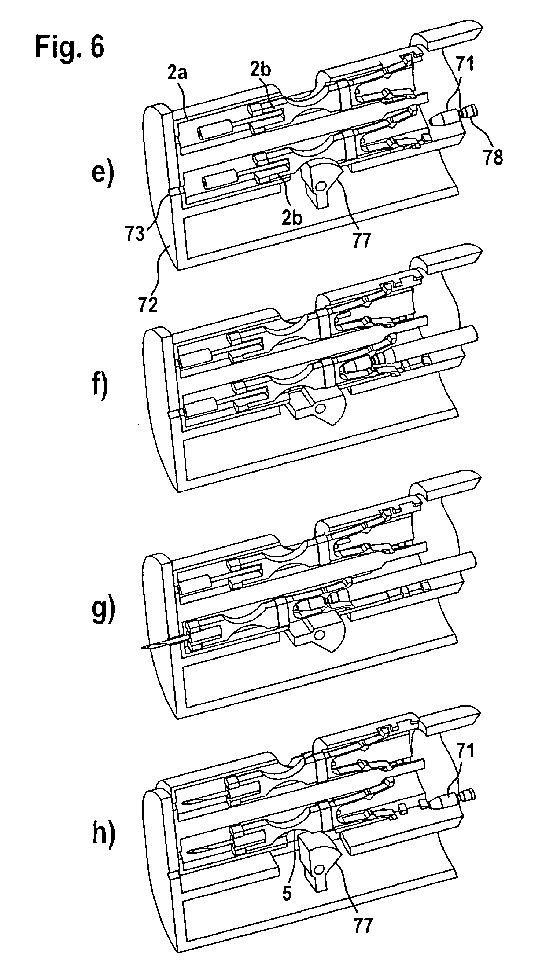

Figur 6 zeigt eine weitere Ausführungsform eines Lanzettensystems, das innerhalb einer Stechhilfe angeordnet ist. Figur 6 a bis d zeigt zunächst ein zu Figur 1 analoges Lanzettensystem. Gemäß Figur 1 verfügt das in Figur 6 gezeigte System ebenfalls über ein Elastomer (4), das die Nadelspitze steril umgibt, sowie einen zweiteiligen Nadelkörper, der in seiner Mitte eine Verjüngung (5, 5') aufweist. Der zweite Teil des Nadelkörpers (2b), der im Inneren des Schutzbereiches des Nadelkörpers (2a) beweglich geführt wird, verfügt ebenfalls in seinem hinteren Bereich über Arme (6), die mit einem Stößel (78) der Stechhilfe (72) formschlüssig ankoppeln können. Wie in Figur 6b und c gezeigt, greift hierfür der Stößel (78) mit einem Kopf (71) in den zweiten Teil (2b) des Nadelkörpers ein und bewegt die Nadel entlang der Achse (8) in Einstichrichtung. Dabei werden die Arme (6) des Nadelkörpers (2b) zusammengedrückt, wobei die Vorsprünge (11) hinter die Einkerbungen des Kopfes (71) greifen. Das Lanzettensystem ist in seiner Funktionsweise, wie bereits in Figur 1 beschrieben, analog gestaltet und wird somit an dieser Stelle lediglich im Zusammenwirken mit der Stechhilfe nochmals dargestellt. Die Stechhilfe (72) verfügt über einen Sperrhebel (74), der in der Stechhilfe montiert ist und auf einer Achse (75) drehbar gelagert wird. Der Sperrhebel (74) ist in einem ersten Bereich (77) kreisförmig ausgebildet, so dass der Sperrhebel passgenau in die Verjüngung (5) des Lanzettensystems eingreift.

Figur 6a zeigt den Zustand der Stechhilfe mit Lanzettensystem vor deren Gebrauch im eingelegten Zustand. Soll die Stechhilfe für ein Stechvorgang verwendet werden, wird der Sperrhebel (74) entweder automatisch beim Auslösen eines ersten Stechvorgangs oder separat durch den Benutzer um seine Achse (75) um ca. 90° gedreht, so dass der untere Bereich (77) des Sperrhebels nicht mehr länger in die Verjüngung (5, 5') eingreift. Damit ein Drehen des Sperrhebels gewährleistet werden kann, verfügt die Stechhilfe weiterhin über eine Mulde (76) im Gehäuse der Stechhilfe, die dem Sperrhebel eine Rotationsbewegung um die Drehachse (75) erlaubt. Beim Ausführen des Stechvorgangs kann nun der zweite Teil des Nadelkörpers (2b) entlang des Schutzbereiches des Nadelkörpers (2a) bewegt werden, wobei ein Bereich des Nadelkörpers (2b) aus der Öffnung des Nadelkörpers (2a) herausragt, ohne jedoch dabei durch den Sperrhebel (74) behindert zu werden. Die Nadelspitze wird hierbei durch das Elastomer und die Austrittsöffnung (9) des Lanzettensystems sowie eine Austrittsöffnung (73) der Stechhilfe hinausgetrieben.

Nach dem Stechvorgang kehrt die Nadel in ihre Ruheposition zurück, wobei sie jedoch mit dem zweiten Teil des Nadelkörpers in die Ausnehmung (13) des Schutzbereiches des Nadelkörpers einrastet. Wie bereits bei Figur 1 beschrieben, verändert hierdurch der Nadelkörper im Bereich der Verjüngung (5) seine äußere Form, da nun der zweite Teil des Nadelkörpers (2b) durch die Öffnung des Nadelkörpers (2a) herausragt. Nach Entfernen des Lanzettensystems aus der Stechhilfe dreht sich der Sperrhebel (74) in seiner Ausgangsposition, wie in Figur 6a beschrieben, zurück. Wie in Figur 6d angedeutet, wird nun ein Wiedereinführen des Lanzettensystems in die Stechhilfe durch den Sperrhebel (74) blockiert. Der Sperrhebel (74) kann nicht mehr in die Verjüngung (5) des benutzten Lanzettensystems eingreifen, da die Verjüngung (5) durch den zweiten Teil des Nadelkörpers (2b) zum Teil verschlossen wird. Das Lanzettensystem kann somit nicht an seinen ursprünglichen Ort positioniert werden und entsprechend gehalten werden. Ein Eingreifen des Stößels (78) in das Lanzettensystem ist nicht mehr möglich. Eine formschlüssige Verbindung zwischen dem Kopf (71) und den Vorsprüngen (11) wird verhindert.

Figur 6e-h zeigen Ausführungsformen analog zu Figur 6a - d die kein Teil der Erfindung sind, wobei das Lanzettensystem aus einer Mehrzahl von Nadeln besteht, so dass eine Magazinierung, wie in Figur 2 gezeigt, gegeben ist. Die Funktionsprinzipien sind wie bereits geschildert identisch und lassen sich von dem System mit einer Nadel auf das in Figur 6e-h gezeigt System einfach übertragen. An dieser Stelle soll somit lediglich eine Ausführungsform veranschaulicht werden, die das Wiedereinführen eines Magazins, dessen Nadeln nur teilweise benutzt wurden, erlaubt. Hierbei muss der Benutzer das Magazin so lange relativ zum Stechhilfegehäuse drehen, bis der Sperrhebel (74) in eine Verjüngung (5), die nicht durch einen Nadelkörper (2b) blockiert wird, eingreifen kann. Die so erzielte Positionierung des Lanzettensystems relativ zur Stechhilfe und folglich zum Antriebsstößel gewährleistet, dass ausschließlich eine noch nicht gebrauchte Lanzette für den nächsten Stechvorgang verwendet wird. Ein Weitertakten des Lanzettensystems in nur eine Drehrichtung sowie ein Mechanismus, der maximal eine Umdrehung des Lanzettensystems um 360° erlaubt, können bei Bedarf ergänzt werden, um einen erneuten Gebrauch von bereits verwendeten Stechhilfen zu verhindern.

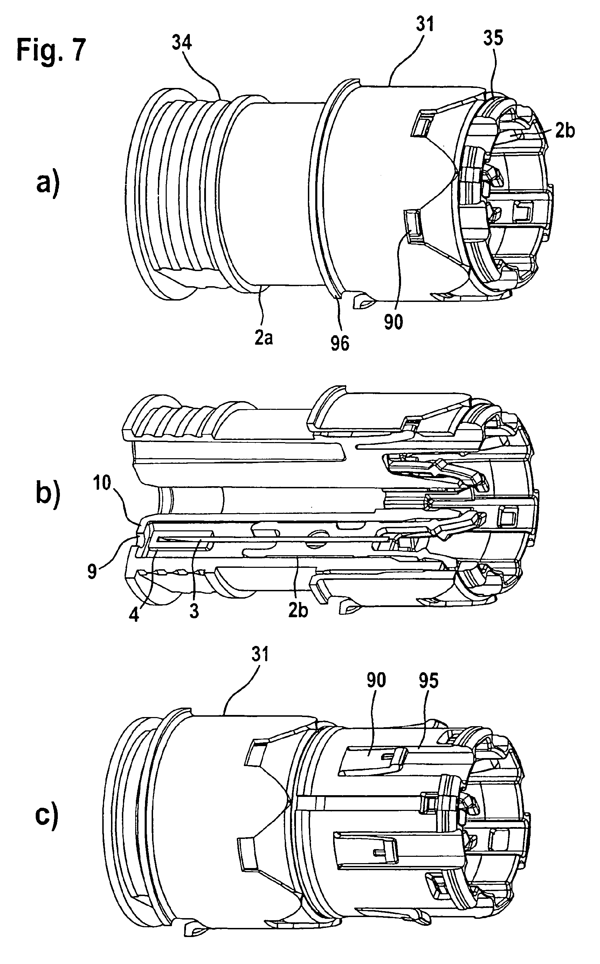

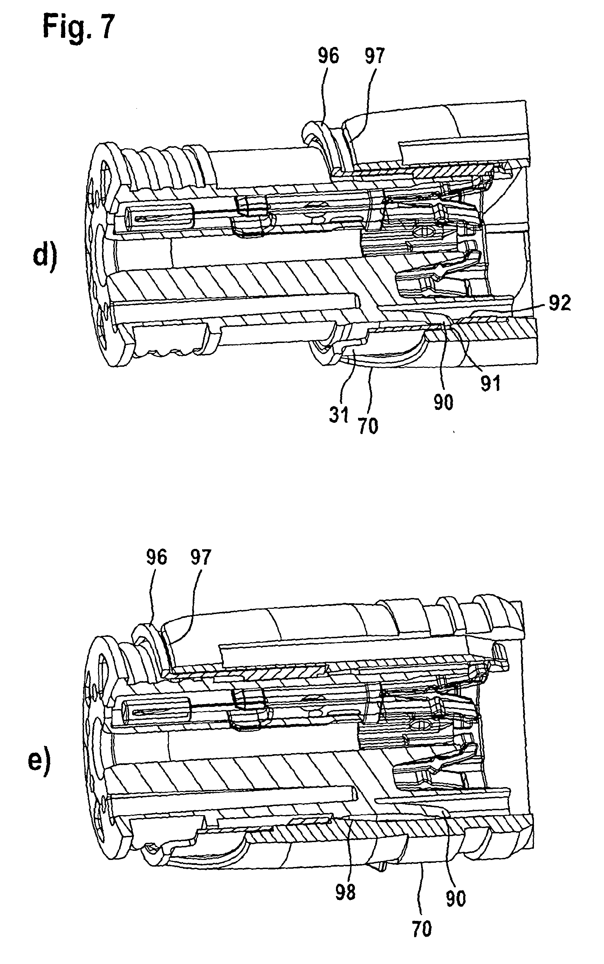

Figur 7 zeigt eine weitere Ausführungsform eines Lanzettensystems, das in Form eines runden Magazingehäuses verwirklicht ist. Der Nadelkörper ist analog zu den bereits beschriebenen Figuren mehrteilig ausgestaltet. Sterilschutz und Nadeln sind ebenfalls, wie bereits beschrieben, angeordnet, sodass an dieser Stelle auf eine weitere detaillierte Beschreibung verzichtet wird. Analog zu dem in Figur 5 beschriebenen System wird das in Figur 7 gezeigte Sperrmechanismus ebenfalls beim Einführen des Lanzettensystems in die Stechhilfe betätigt. Der Nadelkörper (2a) verfügt hierfür über ein Sperrmechanismus (31), der in Form eines äußeren Ringes den oberen Bereich (35) des Nadelkörpers umgibt. An dieser Stelle positioniert, bedeckt der Sperrmechanismus (31) im Wesentlichen elastische Arme (90), die im oberen Bereich des Nadelkörpers (35) angeordnet sind. Hierbei werden die elastischen Arme (90) in Ausnehmung (95) des Nadelkörpers (2a) gedrückt. Der Sperrmechanismus (31) verfügt weiterhin an seinem unteren Ende über eine ringförmig ausgebildete Erhebung, die den Umfang des Nadelkörpers (2a) an dieser Stelle vergrößert. Wird das Lanzettensystem in eine Stechhilfe eingeführt, ist der Umfang der Stechhilfe dabei so gewählt, dass der Ring (96) nicht in das Lanzettensystem eingeführt werden kann. Durch eine untere Kante (97) als Widerlager des Stechhilfengehäuses wird der Ring beim Einschieben des Lanzettensystems in die Stechhilfe relativ zum Nadelkörper (2a) nach unten in den Bereich der Lanzettenspitzen verschoben. Hierbei werden die federgelagerten Rastarme (90) vom Ring freigegeben. Die Rastarme liegen nun in der Stechhilfe in einem gespreizten Zustand vor und werden gegen die innere Gehäusewand (98) der Stechhilfe gedrückt. Beim Entnehmen eines gebrauchten Lanzettensystems aus der Stechhilfe gleiten die Rastarme (90) entlang der schräg ausgebildeten Gehäusewand (98) in einen verjüngten Bereich des Stechhilfegehäuses, wobei sie aufgrund der schräg ausgebildeten Wand (98) zunächst in die Ausnehmung (95) des Nadelkörpers gedrückt werden. Ein Entnehmen des Lanzettensystems aus dem verjüngten Bereich der Stechhilfe ist somit problemlos möglich. Das einmal verwendete Lanzettensystem liegt anschließend, wie in Figur 7c gezeigt, in veränderter Form vor. Beim erneuten Wiedereinführen des Systems in eine Stechhilfe liegen die Rastarme (90) nun im gespreiztem Zustand vor, sodass der Umfang des Nadelkörpers (2a) in den Bereich (35) vergrößert ist. Ein Einschieben des Lanzettensystems in den vorderen, verjüngten Bereich der Stechhilfe wird somit verhindert.

## Patentansprüche

1. Lanzettensystem (1) zum Einführen in eine Stechhilfe (72) beinhaltend
- mindestens eine Nadel (3) mit einer Spitze zum Erzeugen einer Hautöffnung, sowie
- einen Nadelkörper (2a) mit einem Halteelement, das beim Einführen des Lanzettensystems (1) in eine Stechhilfe (72) mit einem Halteelement der Stechhilfe (72) in der Weise zusammenwirkt, dass eine Positionierung des Lanzettensystems (1) an einen definierten Ort in der Stechhilfe(72) erfolgt, und
- der Nadelkörper (2a) mit der Nadel (3) in der Weise verbunden ist, dass zumindest ein Schutzbereich des Nadelkörpers (2a) und die Nadel (3) relativ zueinander beweglich sind, wobei
- in einer ersten Position der Schutzbereich des Nadelkörpers (2a) die Nadelspitze zumindest teilweise umgibt, und
- in einer zweiten Position der Schutzbereich des Nadelkörpers (2a) und die Nadelspitze räumlich voneinander getrennt sind, so dass die Nadelspitze vom Schutzbereich des Nadelkörpers (2a) freigegeben wird,
**dadurch gekennzeichnet, dass**
- der Nadelkörper (2a) weiterhin einen Spernnechanismus (31, 41) beinhaltet, der durch ein Zusammenwirken mit einer Stechhilfe (72) betätigt werden kann und eine Verschiebung von mindestens einem Teil des Nadelkörpers bewirkt, welches so mit der Stechhilfe (72) zusammenwirkt, dass eine Veränderung seiner Position ein Wiederverwenden des Lanzettensystems (1) nach dem Auswurf des Lanzettensystems (1) aus der Stechhilfe (72) verhindert.

2. Lanzettensystem gemäß Anspruch 1,
bei dem der Schutzbereich des Nadelkörpers (2a) ein Magazingehäuse ist, das eine Mehrzahl von Nadeln (3) beinhaltet.

3. Lanzettensystem gemäß Anspruch 1,
bei dem der Nadelkörper (2a) mehrteilig ist.

4. Lanzettensystem gemäß Anspruch 1 oder 3,
bei dem ein Teil des Nadelkörpers (2a) den Sperrmechanismus (31,41) beinhaltet, der unabhängig vom Schutzbereich des Nadelkörpers (2a) betätigt wird.

5. Lanzettensystem gemäß Anspruch 1,
bei dem der Sperrmechanismus (31,41) direkt auf das Halteelement wirkt und das Halteelement bedeckt oder zerstört.

6. Lanzettensystem gemäß Anspruch 1,
bei dem die Form des Nadelkörpers (2a) selbst als Halteelement ausgebildet ist.

7. Lanzettensystem gemäß Anspruch 1,
bei dem der Sperrmechanismus(31,41) eine Sollbruchstelle beinhaltet, die beim Auswurf des Lanzettensystems(1) ein Zerbrechen des Nadelkörpers (2a) bewirkt.

8. Lanzettensystem gemäß Anspruch 1,
bei dem der Sperrmechanismus (31,41) eine Vergrößerung mindestens eines Bereiches des Nadelkörpers (2a) bewirkt.

9. Lanzettensystem gemäß Anspruch 1,
bei dem der Sperrmechanismus (31,41) eine Verkleinerung mindestens eines Bereiches des Nadelkörpers (2a) bewirkt.

10. Lanzettensystem gemäß Anspruch 2, **dadurch gekennzeichnet, dass** der Sperrmechanismus (31) zu einer Verlängerung des Nadelkörpers im Bereich der Nadelspitzen führt, so dass die Nadelspitzen schützend vom Sperrmechanismus (31) umgeben werden.

11. Lanzettensystem gemäß Anspruch 1, **dadurch gekennzeichnet, dass** ein Wiedereinführen des Lanzettensystems (1) in die Stechhilfe (72) nach dem Auswurf des Lanzettensystems (1) aus der Stechhilfe (72) verhindert wird.

12. Stechhilfe (72) zur Erzeugung einer Hautöffnung, beinhaltend
- das Lanzettensystem (1) gemäß einem der Ansprüche 1 bis 9
- ein Gehäuse zum Einführen des Lanzettensystems (1), wobei
- das Gehäuse weiterhin ein Halteelement aufweist, dass mit einem hierzu entsprechenden Halteelement des Lanzettensystems (1) zusammenwirkt, so dass das Lanzettensystem (1) im Gehäuse an einem definierten Ort positioniert wird, und
- das Gehäuse eine Öffnung (73) beinhaltet, aus der eine Nadelspitze mindestens einer Nadel (3) des Lanzettensystems (1) bei einem Stechvorgang hinaustreten kann, sowie
- einen Antriebsmechanismus zum Antreiben der mindestens einen Nadel (3), so dass die Nadel (3) von einer Ruheposition in eine Stechposition überführt wird.

13. Stechhilfe gemäß Anspruch 12,
bei der das Lanzettensystem (1) und die Stechhilfe (72) jeweils über mehrere, voneinander unabhängig wirkende Halteelemente verfügen.

14. Stechhilfe gemäß Anspruch 12,
bei dem der Sperrmechanismus (31,41) eine räumliche Trennung der Halteelemente beim erneuten Wiedereinführen des Lanzettensystems (1) in die Stechhilfe (72) bewirkt.

15. Stechhilfe gemäß Anspruch 12,
bei der der Sperrmechanismus (31,41) bei einem Auswurf des Lanzettensystems (1) aus der Stechhilfe (72) aktiviert wird.

16. Stechhilfe gemäß Anspruch 12,
bei der der Sperrmechanismus (31,41) beim Einführen des Lanzettensystems(1) in die Stechhilfe aktiviert wird.

17. Stechhilfe gemäß Anspruch 12,
bei der der Sperrmechanismus (31,41) bei einem Stechvorgang betätigt wird.

18. Stechhilfe gemäß Anspruch 12,
bei der während des Auswurfs des Lanzettensystems (1) der Schutzbereich des Nadelkörpers (2a) in seine erste Position überführt wird.

19. Stechhilfe gemäß Anspruch 12,
bei der die erste Position des Schutzbereiches des Nadelkörpers (2a) gleich der Ruheposition ist.

## Claims

1. Lancet system (1) for insertion into a lancing aid (72) comprising
- at least one needle (3) with a tip for producing a skin opening and
- a needle body (2a) with a holding element that interacts with a holding element of the lancing aid (72) when the lancet system (1) is inserted into a lancing aid (72) in such a manner that the lancet system (1) is positioned at a defined position in the lancing aid (72) and
- the needle body (2a) is connected with the needle (3) in such a manner that at least one protective portion of the needle body (2a) and the needle (3) can be moved relative to one another
wherein
- the protective portion of the needle body (2a) at least partially surrounds the needle tip in a first position and
- in a second position, the protective portion of the needle body (2a) and the needle tip are spatially separated from one another such that the needle tip is released from the protective portion of the needle body (2a),
**characterized in that**
- the needle body (2a) additionally comprises a blocking mechanism (31, 41) that can be actuated by an interaction with a lancing aid (72) and causes a movement of at least one part of the needle body which interacts with the lancing aid (72) in such a manner that a change in its position prevents a reuse of the lancet system (1) after ejection of the lancet system (1) from the lancing aid (72).

2. Lancet system according to claim 1,
in which the protective portion of the needle body (2a) is a magazine housing that contains a plurality of needles (3).

3. Lancet system according to claim 1,
in which the needle body (2a) comprises several parts.

4. Lancet system according to claim 1 or 3,
in which a part of the needle body (2a) contains the blocking mechanism (31, 41) which is actuated independently of the protective portion of the needle body (2a).

5. Lancet system according to claim 1,
in which the blocking mechanism (31, 41) acts directly on the holding element and covers or destroys the holding element.

6. Lancet system according to claim 1,
in which the shape of the needle body (2a) is itself configured as a holding element.

7. Lancet system according to claim 1,
in which the blocking mechanism (31, 41) comprises a preset breaking point that breaks the needle body (2a) when the lancet system (1) is ejected.

8. Lancet system according to claim 1,
in which the blocking mechanism (31, 41) enlarges at least an area of the needle body (2a).

9. Lancet system according to claim 1,
in which the blocking mechanism (31, 41) reduces at least an area of the needle body (2a).

10. Lancet system according to claim 2, **characterized in that**
the blocking mechanism (31) results in an elongation of the needle body in the area of the needle tips such that the needle tips are surrounded by the blocking mechanism (31) in a protective manner.

11. Lancet system according to claim 1, **characterized in that**
a reinsertion of the lancet system (1) into the lancing aid (72) is prevented after the lancet system (1) has been ejected from the lancing aid (72).

12. Lancing aid (72) for producing an opening in the skin, comprising
- the lancet system (1) according to one of the claims 1 to 9
- a housing for insertion of the lancet system (1), wherein
- the housing additionally has a holding element that interacts with a corresponding holding element of the lancet system (1) such that the lancet system (1) is positioned in the housing at a defined position and
- the housing comprises an opening (73) from which a needle tip of at least one needle (3) of the lancet system (1) can emerge during a lancing operation,
- a drive mechanism for propelling the at least one needle (3) such that the needle (3) is transferred from a resting position into a lancing position.

13. Lancing aid according to claim 12,
in which the lancet system (1) and the lancing aid (72) each have several holding elements that act independently of one another.

14. Lancing aid according to claim 12,
in which the blocking mechanism (31, 41) results in a spatial separation of the holding elements when the lancet system (1) is again reinserted into the lancing aid (72).

15. Lancing aid according to claim 12,
in which the blocking mechanism (31, 41) is activated when the lancet system (1) is ejected from the lancing aid (72).

16. Lancing aid according to claim 12,
in which the blocking mechanism (31, 41) is activated when the lancet system (1) is inserted into the lancing aid.

17. Lancing aid according to claim 12,
in which the blocking mechanism (31, 41) is actuated in a lancing operation.

18. Lancing aid according to claim 12,
in which the protective portion of the needle body (2a) is transferred into its first position during the ejection of the lancet system (1).

19. Lancing aid according to claim 12,
in which the first position of the protective portion of the needle body (2a) is the same as the resting position.

## Revendications

1. Système de lancette (1) destiné à être introduit dans un accessoire de piqûre (72) comprenant
- au moins une aiguille (3) dotée d'une pointe servant à créer une ouverture de la peau, ainsi que
- un corps d'aiguille (2a) doté d'un élément de retenue qui en introduisant le système de lancette (1) dans un accessoire de piqûre (72) coopère avec un élément de retenue de l'accessoire de piqûre (72) de telle manière que le système de lancette (1) se positionne à un endroit défini dans l'accessoire de piqûre (72), et
- le corps d'aiguille (2a) est relié avec l'aiguille (3) de telle manière qu'au moins une zone de protection dudit corps d'aiguille (2a) et ladite aiguille (3) sont mobiles l'une par rapport à l'autre, système dans lequel
- dans une première position, la zone de protection du corps d'aiguille (2a) entoure au moins partiellement la pointe d'aiguille, et
- dans une deuxième position, la zone de protection du corps d'aiguille (2a) et la pointe d'aiguille sont spatialement séparées l'une de l'autre, de sorte que la pointe d'aiguille est libérée par la zone de protection du corps d'aiguille (2a),
**caractérisé en ce que**
- le corps d'aiguille (2a) comprend en outre un mécanisme de blocage (31, 41) pouvant être actionné grâce à une coopération avec un accessoire de piqûre (72) et qui provoque un déplacement d'au moins une partie du corps d'aiguille, laquelle partie coopère avec l'accessoire de piqûre (72) de telle sorte qu'un changement de sa position empêche une réutilisation du système de lancette (1) après l'éjection du système de lancette (1) de l'accessoire de piqûre (72).

2. Système de lancette selon la revendication 1,
dans lequel la zone de protection du corps d'aiguille (2a) est un boîtier de magasin contenant une pluralité d'aiguilles (3).

3. Système de lancette selon la revendication 1,
dans lequel le corps d'aiguille (2a) est en plusieurs parties.

4. Système de lancette selon la revendication 1 ou 3,
dans lequel une partie du corps d'aiguille (2a) comporte le mécanisme de blocage (31, 41) actionné indépendamment de la zone de protection du corps d'aiguille (2a).

5. Système de lancette selon la revendication 1,
dans lequel le mécanisme de blocage (31, 41) agit directement sur l'élément de retenue, et recouvre ou détruit l'élément de retenue.

6. Système de lancette selon la revendication 1,
dans lequel la forme du corps d'aiguille (2a) lui-même se présente sous la forme d'un élément de retenue.

7. Système de lancette selon la revendication 1,
dans lequel le mécanisme de blocage (31, 41) comporte un point de rupture engendrant une rupture du corps d'aiguille (2a) lors de l'éjection du système de lancette (1).

8. Système de lancette selon la revendication 1,
dans lequel le mécanisme de blocage (31, 41) engendre un agrandissement d'au moins une zone du corps d'aiguille (2a).

9. Système de lancette selon la revendication 1,
dans lequel le mécanisme de blocage (31, 41) engendre une réduction d'au moins une zone du corps d'aiguille (2a).

10. Système de lancette selon la revendication 2,
**caractérisé en ce que** le mécanisme de blocage (31) entraîne un allongement du corps d'aiguille dans la zone des pointes d'aiguilles, de telle sorte que les pointes d'aiguilles seront entourées et protégées par le mécanisme de blocage (31).

11. Système de lancette selon la revendication 1,
**caractérisé en ce qu'**une réintroduction du système de lancette (1) dans l'accessoire de piqûre (72) est empêchée après l'éjection du système de lancette (1) de l'accessoire de piqûre (72).

12. Accessoire de piqûre (72) servant à créer une ouverture de la peau, comprenant
- le système de lancette (1) selon l'une des revendications 1 à 9,
- un boîtier servant à introduire le système de lancette (1),
- ledit boîtier comportant en outre un élément de retenue qui coopère avec un élément de retenue correspondant du système de lancette (1), de telle sorte que le système de lancette (1) se positionne à un endroit défini dans le boîtier, et
- ledit boîtier comprenant une ouverture (73) de laquelle peut sortir une pointe d'aiguille d'au moins une aiguille (3) du système de lancette (1) lors d'un processus de piqûre, ainsi que
- un mécanisme d'entraînement de ladite au moins une aiguille (3), permettant ainsi de faire passer ladite aiguille d'une position de repos dans une position de piqûre.

13. Accessoire de piqûre selon la revendication 12,
dans lequel le système de lancette (1) et l'accessoire de piqûre (72) disposent chacun de plusieurs éléments de retenue agissant indépendamment l'un de l'autre.

14. Accessoire de piqûre selon la revendication 12,
dans lequel le mécanisme de blocage (31, 41) engendre une séparation spatiale des éléments de retenue lorsque l'on réintroduit le système de lancette (1) dans l'accessoire de piqûre (72).

15. Accessoire de piqûre selon la revendication 12,
dans lequel le mécanisme de blocage (31, 41) est activé lorsque le système de lancette (1) est éjecté de l'accessoire de piqûre (72).

16. Accessoire de piqûre selon la revendication 12,
dans lequel le mécanisme de blocage (31, 41) est activé lorsque le système de lancette (1) est introduit dans l'accessoire de piqûre (72).

17. Accessoire de piqûre selon la revendication 12,
dans lequel le mécanisme de blocage (31, 41) est actionné lors d'un processus de piqûre.

18. Accessoire de piqûre selon la revendication 12,
dans lequel la zone de protection du corps d'aiguille (2a) est amenée dans sa première position pendant l'éjection du système de lancette (1).

19. Accessoire de piqûre selon la revendication 12,
dans lequel la première position de la zone de protection du corps d'aiguille (2a) est identique à la position de repos.
